# EUROPEAN PATENT APPLICATION

(11) **EP 4 722 722 A1**
(43) Date of publication of application: **08.04.2026**
(21) Application number: 25206429.0
(22) Date of filing: 02.10.2025
(51) Int. Cl.: G01N 33/543

(54) **SENSOR FOR THE DETECTION OF NANOPLASTICS**

(30) Priority: 04.10.2024 KR 20240134771
(71) Applicant: Pusan National University Industry-University Cooperation Foundation, Busan 46241 (KR)
(72) Inventor: LEE, Eun-Hee, Busan (KR); CHANG, Yunsoo, Busan (KR)
(74) Representative: Ter Meer Steinmeister & Partner

(57) **Abstract**

Disclosed is a sensor for detection of nanoplastics, including: a membrane; a sample pad, a conjugate pad and an absorbent pad sequentially arranged on the membrane; and a test line and control line disposed between the conjugate pad and the absorbent pad, wherein the conjugate pad includes a fluorescent dye that binds to a target material, a first polymer that binds to the target material is immobilized on the test line, and a second polymer that binds to the fluorescent dye is immobilized on the control line.

## Description

### CROSS-REFERENCE TO RELATED APPLICATION(S)

This application claims priority from and the benefit of Korean Patent Application No. 10-2024-0134771, filed on October 4, 2024, which is hereby incorporated by reference for all purposes as if set forth herein.

### BACKGROUND

### 1. Technical Field

The present disclosure relates to a sensor for detection of nanoplastics, and more specifically, to a sensor for lateral flow assay-based detection of nanoplastics.

### 2. Related Art

Recently, there have been increasing concerns about the existence and impact of nanoplastics in the fields of environmental protection and public health. Nanoplastics are defined as synthetic polymer particles having a size of 1 to 1,000 nanometers (nm) or 1 to 100 nanometers (nm), and are divided into primary nanoplastics intentionally produced and secondary nanoplastics resulting from physical, chemical, or biological processes. These particles are not removed by water treatment facilities and may enter the environment, where they may be ingested by living organisms and transferred to higher predators through the food chain, potentially affecting humans.

Due to their small size, nanoplastics can easily pass through biological barriers, and thus penetrate deep into cells, causing cellular stress, inflammatory responses, endocrine disruption, and DNA damage. Furthermore, it has been found that nanoplastics can adsorb various harmful substances, such as heavy metals, and thus act as mediators that transport these substances into living organisms.

Current methods for detecting nanoplastics include pyrolysis-gas chromatography-mass spectrometry (Py-GC/MS), scanning electron microscopy (SEM), and the like, but each method has several limitations. Pyrolysis-gas chromatography-mass spectrometry requires complex sample pretreatment, expensive equipment, and complex processes, which make it difficult to achieve on-site real-time detection. Scanning electron microscopy requires difficult sample preparation and treatment, has limited equipment portability and accessibility, and requires specialized skills.

Therefore, there is a need for research and development of a nanoplastic detection method capable of quick, accurate, and convenient measurement.

### SUMMARY

An object of the present disclosure is to provide a sensor for detection of nanoplastics that may detect nanoplastics in a sample with high accuracy and low cost while overcoming the limitations of on-site detection encountered in conventional known methods.

Another object of the present disclosure is to provide a method of detecting nanoplastics using the sensor for detection of nanoplastics.

The problems to be solved by the present disclosure are not limited to the problem(s) mentioned above, and other problem(s) not mentioned will be clearly understood by those skilled in the art from the following description.

To achieve the above objects, the present disclosure provides a sensor for lateral flow assay-based on-site detection of nanoplastics, comprising: a membrane; a sample pad, a conjugate pad and an absorbent pad sequentially arranged on the membrane; and a test line and control line disposed between the conjugate pad and the absorbent pad, wherein the conjugate pad comprises a fluorescent dye that binds to a target material, a first polymer that binds to the target material is immobilized on the test line, and a second polymer that binds to the fluorescent dye is immobilized on the control line.

The test line and the control line may be disposed on the membrane sequentially in a direction from the conjugate pad to the absorbent pad.

The fluorescent dye may comprise at least one selected from the group consisting of polyarylene ether-based fluorescent dyes, pyrene-based fluorescent dyes, stilbene-based fluorescent dyes, rhodamine-based fluorescent dyes, oxazone-based fluorescent dyes, and combinations thereof.

The fluorescent dye may comprise at least one selected from the group consisting of tetraphenylethylene, 1-pyrenebutyric acid N-hydroxysuccinimidyl ester, 4-dimethylamino-4'-nitrostilbene, rhodamine B, Nile Red, Nile Blue, and combinations thereof.

The conjugate pad may further comprise a surfactant.

The surfactant may comprise at least one selected from the group consisting of anionic surfactants, cationic surfactants, nonionic surfactants, amphoteric surfactants, and combinations thereof.

The surfactant may comprise at least one selected from the group consisting of sodium dodecyl sulfate, sodium lauryl sulfate, cetyltrimethylammonium bromide, Triton X-100, Tween-20, Tween-80, cocamidopropyl betaine, and combinations thereof.

The polymer may comprise at least one selected from the group consisting of synthetic polymers, naturally occurring polymers, conductive polymers, molecularly imprinted polymers, and combinations thereof.

The polymer may comprise at least one selected from the group consisting of polyacrylamide, polyethyleneimine, polyallylamine hydrochloride, chitosan, polyaniline, polythiophene, and combinations thereof.

The membrane may comprise at least one selected from the group consisting of nitrocellulose, polyethersulfone, polyethylene, nylon, polyvinylidene fluoride, polyester, polypropylene, and combinations thereof.

The detection of the target material may be performed by performing qualitative analysis to confirm the presence of the target material based on whether the test line develops color, and performing quantitative analysis to confirm the amount of the target material by measuring a signal of the fluorescent dye.

The present disclosure also provides a method of detecting a target material using the lateral flow assay-based sensor for on-site detection of nanoplastics, comprising steps of: introducing a sample containing a target material into the sample pad; and measuring a signal generated at the test line.

The sensor for detection of nanoplastics according to the present disclosure may accurately measure the concentration of nanoplastics in the particle concentration range of 10⁸ to 10¹⁰, and achieve low cost, rapidity, and convenience, so that the sensor may be applied to on-site detection.

The effects of the present disclosure are not limited to the above-described effects, and should be understood to include all effects that may be inferred from the detailed description of the present disclosure or the spirit of the present disclosure as described in the claims.

### BRIEF DESCRIPTION OF THE DRAWINGS

Fig. 1 is a schematic view of a lateral flow assay (LFA)-based sensor for detection of nanoplastics.
Fig. 2A is images showing the detection results and signal intensities when different membranes are used.
Fig. 2B is a graph showing the detection results and signal intensities when different membranes are used.
Fig. 3 shows the results of signal generation from the control line depending on the polystyrene size.
Fig. 4 is images showing the results of LFA depending on the surfactant concentration in a PBN solution.
Fig. 5 is images showing the results of LFA depending on the distance between the test line and the control line.
Fig. 6A is images showing signal changes depending on the PBN concentration when the surfactant concentration is 2%.
Fig. 6B is a graph showing signal changes depending on the PBN concentration when the surfactant concentration is 2%.
Fig. 7A is images showing signal changes depending on the surfactant concentration in 0.015% PBN solution.
Fig. 7B is a graph showing signal changes depending on the surfactant concentration in 0.015% PBN solution.
Fig. 8A is images showing the results of detecting 80 nm polystyrene nanoplastics by LFA.
Fig. 8B is a graph showing the results of detecting 80 nm polystyrene nanoplastics by LFA.
Fig. 9A is images showing the results of detecting 100 nm polystyrene nanoplastics by LFA.
Fig. 9B is a graph showing the results of detecting 100 nm polystyrene nanoplastics by LFA.
Fig. 10A is images showing the results of detecting low-density polyethylene nanoplastics by LFA.
Fig. 10B is a graph showing the results of detecting low-density polyethylene nanoplastics by LFA.
Fig. 11A is images showing the results of detecting polyvinyl chloride nanoplastics by LFA.
Fig. 11B is a graph showing the results of detecting polyvinyl chloride nanoplastics by LFA.
Fig. 12A is images showing the results of detecting polypropylene nanoplastics by LFA.
Fig. 12B is a graph showing the results of detecting polypropylene nanoplastics by LFA.
Fig. 13A is images showing the results of detecting polyethylene terephthalate nanoplastics by LFA.
Fig. 13B is a graph showing the results of detecting polyethylene terephthalate nanoplastics by LFA.
Fig. 14A shows the results of detection by LFA when the interfering agent is bacteria (*Escherichia coli*)*.*
Fig. 14B shows the results of detection by LFA when the interfering agent is protein (BSA).
Fig. 14C shows the results of detection by LFA when the interfering agent is other nano-sized material (gold nanoparticles).
Fig. 14D shows the results of detection by LFA when the interfering agent is an organic matter (humic acid).
Fig. 15A is images showing the results of detecting 100 nm polystyrene nanoplastics in each actual sample by LFA.
Fig. 15B is a graph showing the results of detecting 100 nm polystyrene nanoplastics in each actual sample by LFA.
Fig. 16A is images showing the results of comparing the performance between the portable fluorescence measurement device and the laboratory fluorescence measurement device (UV lamp).
Fig. 16B is a graph showing the results of comparing the performance between the portable fluorescence measurement device and the laboratory fluorescence measurement device (UV lamp).
Fig. 17A is images showing the results of detecting a nanoplastic mixture sample in each environmental sample by LFA.
Fig. 17B is a graph showing the result of detecting a nanoplastic mixture sample in each environmental sample by LFA.
Fig. 18A is images showing the results of analyzing 10¹⁰ particles of 100 nm polystyrene nanoplastics using strips stored for a certain period of time under each storage condition.
Fig. 18B is a graph showing the result of analyzing 10¹⁰ particles of 100 nm polystyrene nanoplastics using strips stored for a certain period of time under each storage condition.

### DETAILED DESCRIPTION

It should be noted that, in the following description, only the parts necessary for understanding embodiments of the present disclosure are described, and the description of other parts may be omitted when it may unnecessarily obscure the subject matter of the present disclosure.

The terms or words used in the specification and claims should not be construed as being limited to typical meanings or dictionary definitions, but should be construed as having meanings and concepts relevant to the technical ideas of the present disclosure based on the principle that the inventor can appropriately define the meaning of the terms to describe his/her own invention in the best manner. Accordingly, it should be understood that the embodiments described in the specification and the configurations shown in the drawings are merely preferred examples of the present disclosure, but do not cover all the technical spirits of the present disclosure, and thus there may be various equivalents and modifications capable of replacing them at the time of filing of the present disclosure.

The inventors of the present disclosure have paid attention to the need for a new technology capable of detecting nanoplastics in a user-friendly, rapid, and accurate manner on-site, and have developed a lateral flow assay (LFA)-based sensor for detection of nanoplastics, thereby completing the present disclosure.

In the present disclosure, the lateral flow assay-based sensor for detection of nanoplastics is also referred to as "LFA sensor", "LFA-based sensor" or "LFA-based sensor for detection of nanoplastics".

One embodiment of the present disclosure provides a sensor for on-site detection of nanoplastics, including: a membrane; a sample pad, a conjugate pad and an absorbent pad sequentially arranged on the membrane; and a test line and control line disposed between the conjugate pad and the absorbent pad, wherein the conjugate pad includes a fluorescent dye that binds to a target material, a first polymer that binds to the target material is immobilized on the test line, and a second polymer that binds to the fluorescent dye is immobilized on the control line.

In one embodiment of the present disclosure, the first polymer and the second polymer, which are immobilized on the test line and the control line, respectively, may have different chemical structures, and the polymers may be designed to selectively bind to a target material or a fluorescent dye, respectively. The first polymer has an affinity for the target material and can selectively capture the target material in a sample passing through the test line, thereby confirming the presence of the target material. On the other hand, the second polymer may act as a reference to determine whether the sensor is functioning normally by binding to the fluorescent dye.

In one embodiment of the present disclosure, the first polymer and the second polymer, which are immobilized on the test line and the control line, respectively, may have the same chemical structure, and may be designed so that they may exhibit different bonding characteristics even when the first polymer and the second polymer have the same chemical structure. In these cases, even if the same polymer is used, the functions are separated such that the polymer selectively binds to the target material in the test line and binds to the fluorescent dye in the control line. To this end, the surface functional groups or arrangements of the polymer may be designed differently, or the binding affinity may be adjusted so that the same polymer may recognize different binding targets in each line. Furthermore, even if the same polymer is used, it may selectively bind to the target material or fluorescent dye in each line by adjusting the concentration or arrangement of the polymer.

As used herein, the term "target material" refers to a target material to be analyzed for its concentration or presence, and may mean, for example, a nanoplastic which is at least one material selected from the group consisting of polyethylene, polyethylene terephthalate, polyvinyl chloride, polyamide, polypropylene, polystyrene, polycarbonate, and combinations thereof, without being limited thereto.

As used herein, the term "sample" may mean a material suspected of containing the target material to be analyzed.

The membrane may be formed to comprise at least one selected from the group consisting of nitrocellulose, polyethersulfone, polyethylene, nylon, polyvinylidene fluoride, polyester, polypropylene, and combinations thereof, without being limited thereto.

The pore size of the membrane may be 1 nm to 10,000 nm, 3,000 nm to 5,0000 nm, 5,000 nm to 8,000 nm, 9,000 nm to 12,000 nm, 10,000 nm to 15,000 nm, 12,000 nm to 17,000 nm, or 9,000 nm to 12,000 nm. When the pore size of the membrane is satisfied, a signal indicating the detection of nanoplastics may be well confirmed.

The sample pad may be positioned at or adjacent to the end of the membrane. The sample pad may accommodate a fluid sample moving through the membrane. The primary function of the sample pad is to facilitate the capillary action of the fluid moving through the membrane and assist in the diffusive flow of the fluid. This ensures efficient operation of the entire system and may play an important role in enhancing the stability and reliability of the analysis process.

The sample pad may be composed of a porous material to effectively capture and move nanoplastics in a fluid sample. Such a porous material may include at least one selected from the group consisting of fibrous paper, microporous membranes made of cellulose materials, cellulose, cellulose derivatives such as cellulose acetate, nitrocellulose, glass fibers, naturally occurring cotton, fabrics such as nylon, polyester, porous gels, and combinations thereof, without being limited thereto.

The conjugate pad may accommodate a sample that moves by diffusion from the sample pad. The conjugate pad may include a fluorescent dye that binds to a target material and may be designed so that the sample and the fluorescent dye may interact with each other. The conjugate pad may be composed of a material that enables diffusive flow, similar to the sample pad.

The conjugate pad may include at least one selected from the group consisting of glass fiber, polyester, cellulose, polypropylene, nylon, and combinations thereof, without being limited thereto.

The fluorescent dye included in the conjugate pad refers to a dye that acts as a detectable label. Any fluorescent material that may be used for plastic detection may be used as the fluorescent dye.

In one embodiment, the composition may include a dye that emits fluorescence at a wavelength of 200 to 800 nm.

In one embodiment, the fluorescent dye may include at least one selected from the group consisting of polyarylene ether-based fluorescent dyes (e.g., tetraphenylethylene, etc.), pyrene-based fluorescent dyes (e.g., 1-pyrenebutyric acid N-hydroxysuccinimidyl ester, etc.), stilbene-based fluorescent dyes (e.g., 4-dimethylamino-4'-nitrostilbene, etc.), rhodamine-based fluorescent dyes (e.g., rhodamine B, etc.), oxazone-based fluorescent dyes (e.g., Nile Red , Nile Blue, etc.), and combinations thereof, without being limited thereto.

In one embodiment, the fluorescent dye may be a pyrene-based fluorescent dye that may detect as many types of plastics as possible, is well released from the conjugate pad, and has excellent fluidity in the membrane.

The conjugate pad may include a surfactant to facilitate diffusion of the fluorescent dye and prevent nonspecific binding other than binding to the target material.

The surfactant may include at least one selected from the group consisting of anionic surfactants (e.g., sodium dodecyl sulfate, sodium lauryl sulfate, etc.), cationic surfactants (e.g., cetyltrimethylammonium bromide, etc.), nonionic surfactants (e.g., Triton X-100, Tween-20, Tween-80, etc.), amphoteric surfactants (e.g., cocamidopropyl betaine), and combinations thereof, without being limited thereto.

In one embodiment, the surfactant may be a nonionic surfactant to improve the dispersion and fluidity of the sample.

The surfactant may be included in an amount of 0.001 to 10 wt%, 0.001 to 9 wt%, 0.001 to 8 wt%, 0.001 to 7 wt%, 0.001 to 6 wt%, 0.001 to 5 wt%, 0.001 to 4 wt%, 0.001 to 3 wt%, 0.01 to 3 wt%, 0.1 to 3 wt%, or 1 to 3 wt%, based on the total weight of the solution included in the conjugate pad. When the surfactant is included in an amount within the above-described range, there is an effect in that the sample binds well to the fluorescent dye and has stable dispersibility and fluidity.

The fluorescent dye may be included in an amount of 0.0001 to 10 wt%, 0.0001 to 9 wt%, 0.0001 to 8 wt%, 0.0001 to 7 wt%, 0.0001 to 6 wt%, 0.0001 to 5 wt%, 0.0001 to 4 wt%, 0.0001 to 3 wt%, 0.0001 to 2 wt%, 0.0001 to 1 wt%, 0.001 to 1 wt%, 0.01 to 1 wt%, or 0.01 to 0.1 wt%, based on the total weight of the solution included in the conjugate pad. When the fluorescent dye is included in an amount within the above-described range, there is an effect in that the sample binds well to the fluorescent dye and has stable dispersibility and fluidity.

In one embodiment, the test line and the control line may be disposed on the membrane sequentially in a direction from the conjugate pad to the absorbent pad.

A first polymer that binds to a target material is immobilized on the test line in order to detect whether the target material is present. The first polymer may include at least one selected from the group consisting of synthetic polymers (e.g., polyacrylamide, polyethyleneimine, polyallylamine hydrochloride, etc.), naturally occurring polymers (e.g., chitosan, etc.), conductive polymers (e.g., polyaniline, polythiophene, etc.), molecularly imprinted polymers, and combinations thereof, without being limited thereto.

The first polymer may be included in an amount of 0.001 to 10 wt% based on the total weight of the solution included in the test line. When the first polymer is included in an amount within the above-described range, the target material may be sensitively detected and the specificity for the target material may be increased. The test line may be prepared using a solution including a solvent in addition to the first polymer.

The solvent included in the solution used for the test line may include at least one selected from the group consisting of halogenated hydrocarbon-based solvents (e.g., chloroform, dichloromethane, trichloroethylene, etc.), alcohol-based solvents (e.g., methanol, ethanol, isopropanol, butanol, etc.), ketone-based solvents (e.g., acetone, methyl ethyl ketone, cyclohexanone, etc.), ester-based solvents (e.g., ethyl acetate, butyl acetate, etc.), amine-based solvents (e.g., triethylamine, diethylamine, etc.), amide-based solvents (e.g., dimethylformamide (DMF), dimethylacetamide (DMAc), etc.), acetic acid-based solvents (e.g., acetic acid, trifluoroacetic acid, etc.), ether-based solvents (e.g., diethyl ether, tetrahydrofuran (THF), etc.), aromatic hydrocarbon-based solvents (e.g., benzene, toluene, xylene, etc.), nitrile-based solvents (e.g., acetonitrile, propionitrile, etc.), and combinations thereof, without being limited thereto.

In one embodiment, the first polymer may be immobilized on the membrane in an amount of 0.1 to 20 µL/cm. When the first polymer is immobilized in an amount within the above-described range, the detection sensitivity may be improved.

The control line serves to check whether the detection system is functioning properly, and a second polymer that may bind to a fluorescent dye and generate a detection signal therefor is immobilized thereon. Any material that may bind to a fluorescent dye may be used as the second polymer.

In one embodiment, the second polymer may include at least one selected from the group consisting of polyethylene, polyethylene terephthalate, polyvinyl chloride, polyamide, polypropylene, polystyrene, polycarbonate, and combinations thereof, without being limited thereto.

In one embodiment, the second polymer may use surface properties, including, but not limited to, surface charge (e.g., cationic, anionic, nonionic, etc.), hydrophilicity, hydrophobicity, and surface functionalization (e.g., amino-functionalized polymer, carboxyl-functionalized polymer, etc.), to bind to the fluorescent dye.

The second polymer may be included in an amount of 0.01 to 10 wt% by weight based on the total weight of the solution included in the control line.

The second polymer may be in the form of particles, and the second polymer particles may have an average diameter of 1 to 1,000 nm, or 1 to 100,000 nm. When the average diameter of the second polymer is within the above range, there is an effect in that the signal of the control line may be kept constant, thereby checking whether the sensor is functioning normally, and the reliability of the results is increased.

In one embodiment, various functional groups may be introduced to the surface of the second polymer particles. Such functional groups may include, but are not limited to, at least one selected from the group consisting of a hydroxyl group (-OH), a carboxyl group (-COOH), an amino group (-NH₂), a sulfonyl group (-SO₃H), and combinations thereof.

The control line may be prepared using a solution including a solvent in addition to the second polymer.

The solvent included in the solution used for the control line may include at least one selected from the group consisting of alcohol-based solvents (e.g., methanol, ethanol, isopropanol, etc.), ketone-based solvents (e.g., acetone, methyl ethyl ketone, cyclohexanone, etc.), ester-based solvents (e.g., ethyl acetate, butyl acetate, etc.), ether-based solvents (e.g., diethyl ether, tetrahydrofuran (THF), etc.), halogenated hydrocarbon-based solvents (e.g., dichloromethane, chloroform, etc.), amine-based solvents (e.g., triethylamine, ethylenediamine, etc.), aromatic hydrocarbon-based solvents (e.g., toluene, xylene, etc.), acetamide-based solvents (e.g., N,N-dimethylacetamide, N-methyl-2-pyrrolidone, etc.), and combinations thereof, without being limited thereto.

In one embodiment, a surfactant may be added to the solution to improve the dispersibility of the second polymer (or second polymer particles). The surfactant may include at least one selected from the group consisting of anionic surfactants (e.g., sodium dodecyl sulfate, sodium lauryl sulfate, etc.), cationic surfactants (e.g., cetyltrimethylammonium bromide, etc.), nonionic surfactants (e.g., Triton X-100, Tween-20, Tween-80, etc.), amphoteric surfactants (e.g., cocamidopropyl betaine, etc.), and combinations thereof, without being limited thereto.

The absorbent pad may be positioned at or adjacent to the end of the membrane. The absorbent pad typically accommodates a fluid sample moving throughout the membrane. The absorbent pad may help facilitate the capillary action and diffusive flow of the fluid through the membrane.

The absorbent pad may include at least one selected from the group consisting of cellulose, polyester, rayon, blended fibers, pulp-based fibers, and combinations thereof, without being limited thereto.

The detection of the target material may be performed by performing qualitative analysis to confirm the presence of the target material based on whether the test line develops color, and performing quantitative analysis to confirm the amount of the target material by measuring a signal of the fluorescent dye.

In one embodiment of the present disclosure, a method of detecting a target material using the sensor for detection of nanoplastics is provided.

For the detection of the target material, qualitative analysis and quantitative analysis may be simultaneously performed by performing qualitative analysis to confirm the presence of the target material based on the test line develops color, and performing quantitative analysis to confirm the amount of the target material by measuring a signal of the fluorescent dye.

**In** one embodiment, the sensor for detection of nanoplastics may be used to detect nanoplastics in seawater or freshwater.

Although the technical idea of the present disclosure has been described above with reference to the embodiments, those skilled in the art to which the present disclosure pertains will appreciate that various modifications and variations are possible without departing from the essential characteristics of the present disclosure. Therefore, the embodiments described in the present disclosure are not intended to limit the technical idea of the present disclosure, but rather to explain them, and the scope of the technical idea of the present disclosure is not limited by these embodiments. The protection scope of the present disclosure should be defined by the claims, and all technical ideas within the scope equivalent thereto should be construed as being included within the scope of the rights of the present disclosure.

### [Example]

### Materials

1-Pyrenebutyric acid N-hydroxysuccinimidyl ester (PBN), polyethyleneimine, and Triton X-100 were purchased from Sigma-Aldrich (St. Louis, MO, USA). Acetone and ethanol were purchased from DUKSAN PURE CHEMICALS CO. (Gyeonggi-do, Korea). As nitrocellulose membranes, FF120HP and FF170HP were purchased from Whatman (Kent, UK), HF135 and HF180 were purchased from Millipore (Burlington, MA, USA), and UNI180 was purchased from Satorius (Göttingen, Germany). Sample pads (CFSP20300), conjugate pads (GFDX103000), absorbent pads (CF7), and laminated cards (HF000MC100) were obtained from Millipore. 80 nm polystyrene particles and 80 nm carboxylated polystyrene particles were purchased from Spherotech, Inc. (Lake Forest, IL, USA). 100 nm polystyrene particles were purchased from EPRUI Biotech (Shanghai, China).

### Preparation of a solution containing a fluorescent dye (hereinafter also referred to as "PBN solution")

The PBN solution was prepared at a concentration of 0.015% (150 µg/mL) in 2.3% Triton X-100 and 25% acetone. Before use, the solution was filtered using a polytetrafluoroethylene (PTFE) syringe filter (0.22 µm, Whatman, GE Healthcare Life Sciences, Santa Clara, CA, USA).

### Fabrication of Lateral Flow Assay (LFA)-Based Sensor

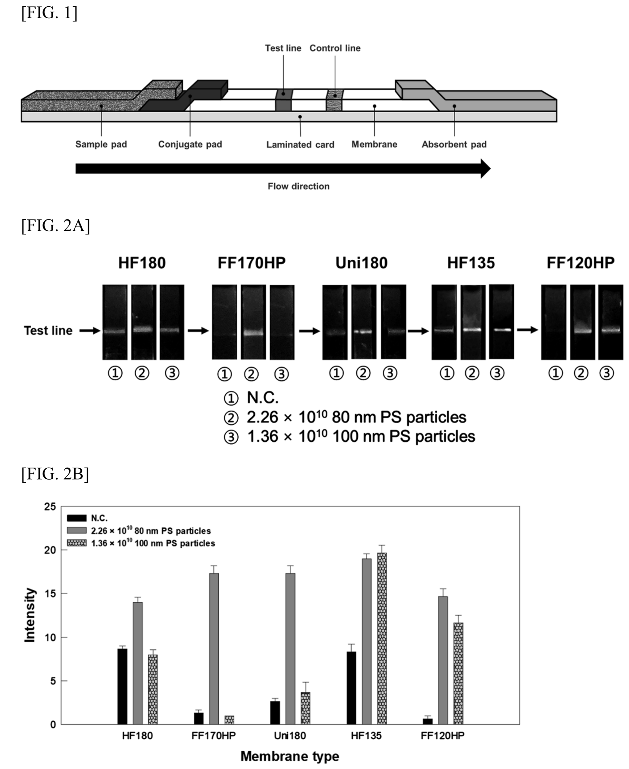

Fig. 1 is a schematic view showing the structure of a LFA-based sensor for detection of nanoplastics. The LFA sensor may include four components: a sample pad, a conjugate pad, a nitrocellulose membrane, and an absorbent pad. The components were fixed onto a laminated card. After attaching the nitrocellulose membrane to the laminated card, a test line and a control line were formed on the membrane. On the test line and the control line, polyethyleneimine and polystyrene particles were immobilized, respectively, using a dispenser (ZX1010, Irvine, CA, USA). After polyethyleneimine (0.5%) was immobilized on the test line on the nitrocellulose membrane in an amount of 0.5 µL/cm, polystyrene particles (2.5%) were subsequently immobilized on the control line. The distance between the test line and the control line was set to about 5 mm. After loading, the membrane was dried at 37°C for 2 hours. The conjugate pad into which the PBN solution has been absorbed was also dried at 37°C for 2 hours. After drying the nitrocellulose membrane, the conjugate pad, the sample pad and the absorbent pad were sequentially assembled onto the laminated card. The components were assembled with an overlap of about 2 mm to facilitate the movement of the solution during the analysis process (Fig. 1).

### Nanoplastic Analysis

Polystyrene nanoplastic particles were dispersed in deionized (DI) water, thereby preparing solutions of varying concentrations, and low-density polyethylene, polyvinyl chloride, polypropylene, and polyethylene terephthalate nanoplastic particles were dispersed in 0.1% Triton X-100 solution, thereby preparing solutions of varying concentrations. The concentration range was set from 1.0×10⁸ to 1.0×10¹⁰ particles. 150 µL of the prepared nanoplastic solution was dispensed into a 96-well plate, and then brought into contact with the LFA sensor and allowed to react for 15 minutes. Thereafter, the results of reaction of the LFA sensor were visualized using a UV lamp in a darkroom. The UV lamp generated a signal from the LFA sensor, allowing for visualization. Fluorescence images were captured using an iPhone 12 Pro Max. The captured images were used to analyze the results of detection of nanoplastics and quantified using GelAnalyzer (Tolima, Colombia, LazarSoftware).

### [Experimental Example]

### 1. Measurement of Change in Detection Signal Depending on Membrane

In the present disclosure, the efficiency of detection of nanoplastics was evaluated using various membranes, and a membrane suitable for nanoplastic detection was selected. Fig. 2 includes images (A) and a graph (B), which show the detection results and signal intensities when different membranes were used. The membranes used in the present disclosure are HF180, HF135, Uni180, FF170HP, and FF120HP, each having a different pore size. The number included in the name of each membrane indicates the pore size of the membrane, and a larger number means a smaller pore size.

According to the experimental results of the present disclosure, the signal intensity generated upon nanoplastic detection tended to increase as the pore size of the membrane was smaller. However, it was observed that, as the particle size of nanoplastics increased, the particles were unable to pass through the pores of the membrane and became blocked, and thus no detection signal was generated. In particular, the HF180 and HF135 membranes showed a false positive signal, and the Uni180 membrane generated a detection signal when loaded with a 80 nm polystyrene nanoplastic sample, but did not detect 100 nm polystyrene nanoplastics. The FF120HP membrane showed lower signal intensity than the FF170HP for 80 nm polystyrene nanoplastics, but showed relatively high detection signal intensity for 100 nm nanoplastics.

**Based** on these experimental results, FF120HP was determined to be the most suitable membrane capable of effectively detecting nanoplastics of various sizes, and thus was chosen for use in the present disclosure.

### 2. Selection of Constituent Material of Control Line

In the present disclosure, carboxylated polystyrene nanoplastics with various particle sizes were considered as candidate materials for constructing the control line. The sizes of these polystyrene nanoplastics as candidates varied at 80 nm, 180 nm, 300 nm, 480 nm, and 1,040 nm. The candidate materials were dispensed at a concentration of 2.5% at 9 mm from the starting point of the membrane, and then evaluated by measuring the signal generated upon loading with DI water.

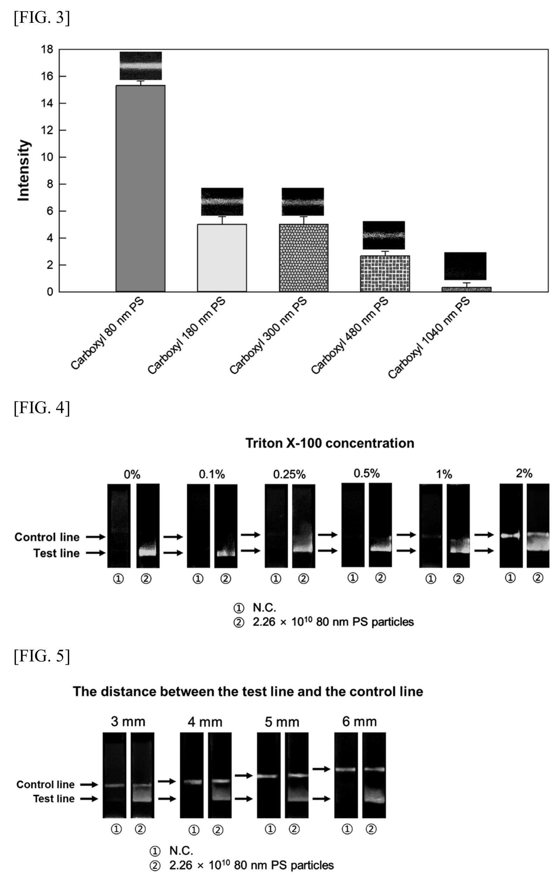

Fig. 3 shows the signal intensity of the control line depending on the polystyrene particle size. Experimentally, it was observed that the LFA signal intensity tended to decrease as the particle size of the nanoplastics used for the control line increased. This could affect the control line's intended function of providing a distinct visual signal. In the present disclosure, it was determined that it was desirable to select polystyrene nanoplastics with small particle sizes in order to maximize signal intensity and the clarity of the control line. Accordingly, 80 nm polystyrene nanoplastics were selected as a constituent material for the control line.

### 3. Changes in Control Line Signal Generation Depending on Concentration of Surfactant (Triton X-100) in PBN Solution

The control line of the LFA according to the present disclosure was constructed using 80 nm carboxylated polystyrene nanoplastics. However, in the above-described experiment, it was observed that no fluorescence signal was generated at the 13-mm point, although the 80 nm carboxylated polystyrene nanoplastics generated a signal by reaction with PBN at the 9-mm point of the membrane. This was believed to be because the PBN solution did not move sufficiently in the LFA and did not reach the control line.

To solve this problem, whether control line signals were generated was investigated while varying the surfactant concentration in the PBN solution. Experiments were conducted at surfactant concentrations set to 0.1%, 0.25%, 0.5%, 1%, and 2%. Fig. 4 includes images showing the results of LFA depending on the surfactant concentration. Experimental results confirmed that a clear signal was generated at the control line when the surfactant concentration reached 2%. This demonstrates that an appropriate surfactant concentration increases the fluidity of PBN, providing sufficient mobility of PBN in the LFA, and is therefore essential for generating a control line signal.

### 4. Optimization of Distance between Test Line and Control Line

In the present disclosure, it was observed that the detection signal range at the test line was expanded by adding a surfactant to the PBN solution. As a result, a phenomenon occurred in which the signal at the test line overlapped with the signal at the control line. This was recognized as a problem that hindered signal differentiation in the LFA, making interpretation of the results difficult.

According to the present disclosure, as a solution to this problem, a method of adjusting the distance between the test line and the control line was introduced. To this end, experiments were conducted while adjusting the distance between the test line and the control line to 3 mm, 4 mm, 5 mm, and 6 mm. Fig. 5 includes images showing the results of LFA depending on the distance between the test line and the control line. Through these experiments, the detection signals at each distance were compared, and the experimental results confirmed that the signals at the test line and the control line did not overlap when the distance was 5 mm.

Therefore, in the present disclosure, the optimal distance between the test line and the control line in the LFA was set to 5 mm in order to solve the problem of detection signal range expansion caused by surfactant addition. This distance setting enables a clear distinction between the two lines, allowing users to interpret assay results more clearly and accurately.

### 5. Optimization of PBN Solution Concentration and Surfactant Concentration

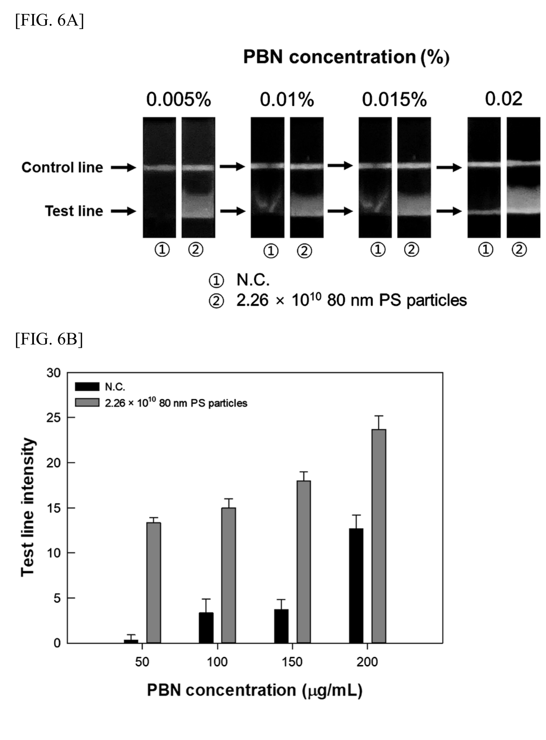

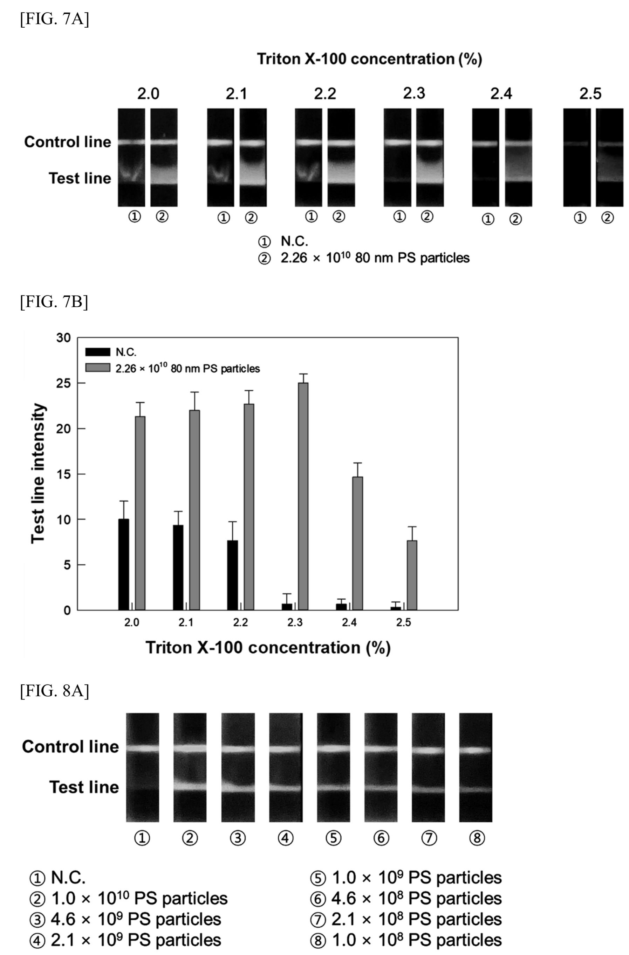

In the present disclosure, optimal conditions were derived by evaluating changes in signal intensity depending on the PBN concentration and surfactant concentration (0.1, 0.25, 0.5, 1, and 2%). Fig. 6 shows images (A) and a graph (B) depicting PBN concentration-dependent signal changes when the surfactant concentration is 2%, and Fig. 7 shows images (A) and a graph (B) depicting signal changes depending on the surfactant concentration in 0.015% PBN solution. When the surfactant concentration was 2%, the signal intensity of the test line increased as the PBN concentration was gradually increased, but a false positive signal appeared at a PBN concentration of 0.02%.

Accordingly, in the present disclosure, an experiment was conducted while adjusting the surfactant concentration between 2.0% and 2.5% at a PBN concentration of 0.015%, excluding a PBN concentration of 0.02% where a false positive signal was observed, and as a result, it was confirmed that the optimal condition for preventing aggregation of PBN was 2.3%. Considering that a decrease in the signal intensity of LFA was observed when the surfactant concentration was 2.4% or higher, it was found that it was preferable to set the surfactant concentration to 2.3%.

Accordingly, the present disclosure provides a method of setting optimal detection conditions in LFA. The method includes maintaining the PBN concentration at 0.015% and setting the surfactant concentration at 2.3%, thereby effectively preventing aggregation of PBN and optimizing the detection signal intensity of LFA.

### 6. Detection of Broad Concentration Range of Nanoplastics

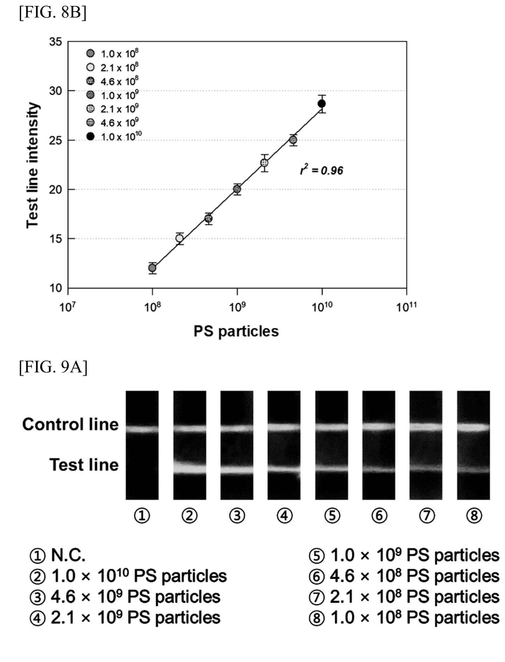

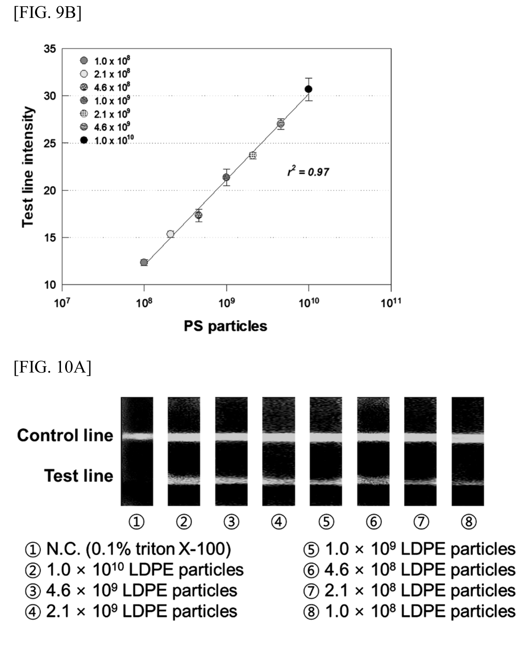

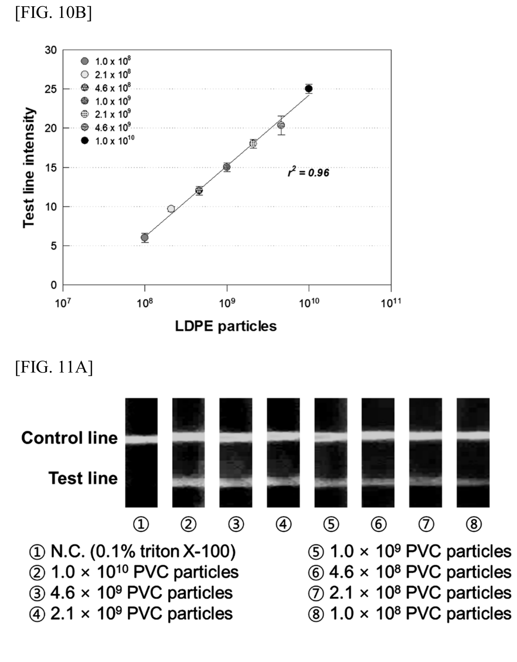

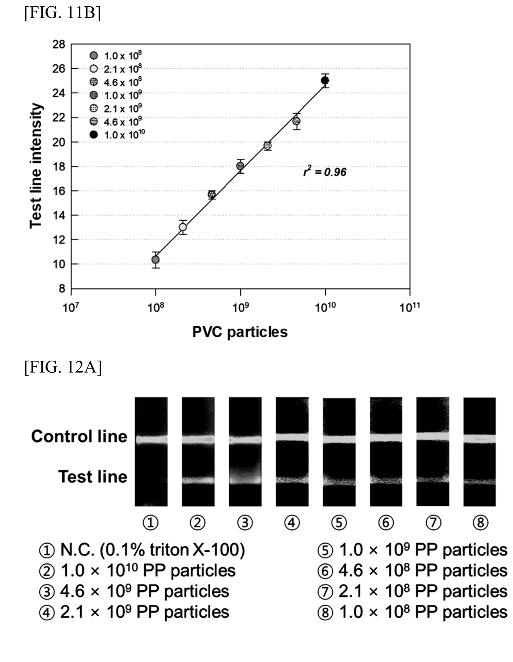

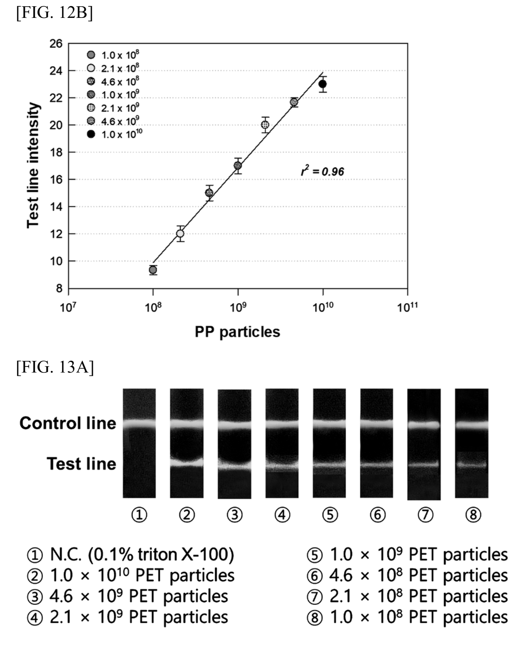

Concentration-dependent detection experiments were conducted on five types of nanoplastics using the sensor for detection of nanoplastics according to the present disclosure. Figs. 8 and 9 show images (A) and graphs (B) depicting the results of detecting 80 nm and 100 nm polystyrene nanoplastics, respectively, by LFA. Figs. 10, 11, 12, and 13 show images (A) and graphs (B) depicting the results of detecting low-density polyethylene, polyvinyl chloride, polypropylene, and polyethylene terephthalate nanoplastics, respectively, by LFA. The experiments were conducted in the set concentration range (1.0 × 10⁸ to 1.0 × 10¹⁰ particles). Experimental results showed that, as the concentration increased, the fluorescence signal at the test line became stronger. This is believed to be because the higher the concentration of nanoplastic particles, the higher the probability of binding of the fluorescent dye-nanoplastic complex at the test line. On the other hand, when the concentration was low, the signal at the test line tended to weaken. This is presumed to be because, at lower concentrations, the nanoplastic-fluorescent dye complex was not sufficiently formed, so that signal expression decreased and the complex relatively less bound to the test line. The above experimental results demonstrate that the sensor of the present disclosure can measure nanoplastics depending on their concentration.

### 7. Evaluation of Specificity of LFA

The present disclosure includes experiments to investigate the effects of various interfering agents that may occur in a detection environment on the detection ability of LFA. The interfering agents selected were bacteria (*Escherichia coli*)*,* protein (BSA), other nano-sized material (gold nanoparticles), and organic matter (humic acid). These interfering agents were considered as important variables in the experiments of the present disclosure because they have the potential to affect the detection signal when they are present together with nanoplastics in the detection environment.

Bacteria are commonly found in detection environments and are biological factors that can interfere with LFA signals. Proteins are abundant in various biological metrices and can cause nonspecific binding in detection systems. Nano-sized materials can be similar in size to the nanoplastics to be detected, potentially interfering with signal differentiation. Organic matter is commonly present in environmental samples and can affect the detection process due to its various chemical properties.

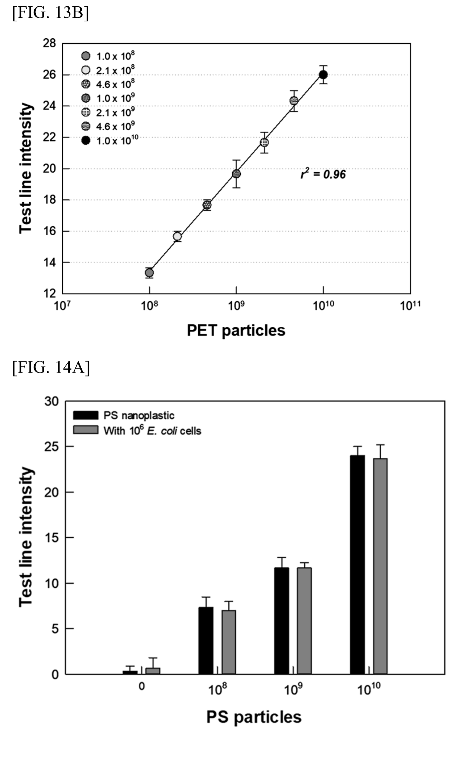

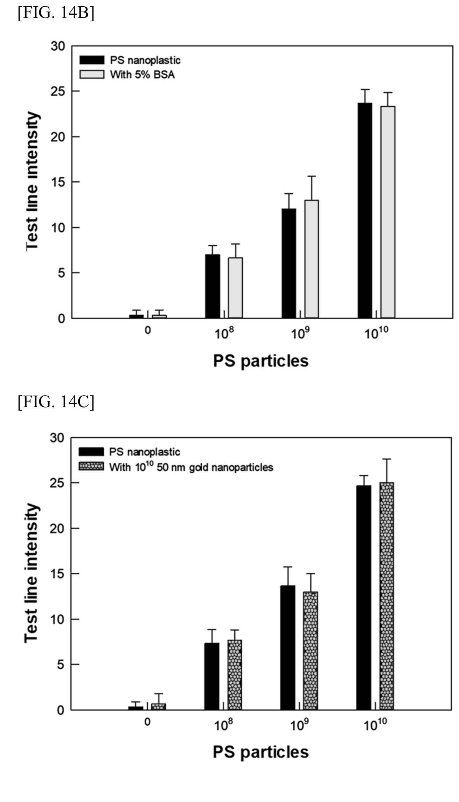

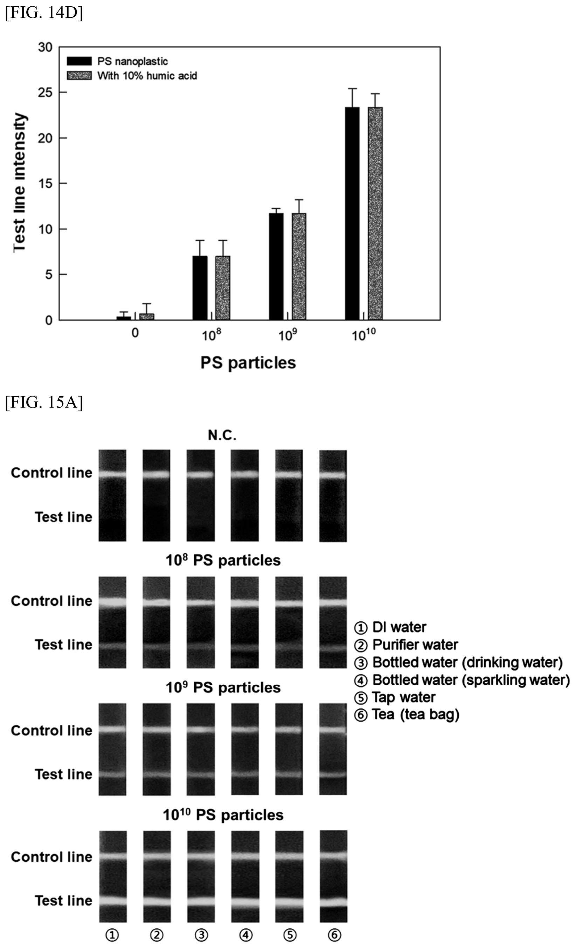

In the experiment according to the present disclosure, the above-described interfering agents were added to LFA, and then the detection signals at different concentrations (10⁸, 10⁹, and 10¹⁰ particles) of 100 nm polystyrene nanoplastics were compared. Fig. 14 shows graphs showing the results of detection by LFA when interfering agents are present. Specifically, Fig. 14(A) shows detection results when the interfering agent is bacteria (*Escherichia coli*)*,* Fig. 14(B) shows detection results when the interfering agent is protein (BSA), Fig. 14(C) shows detection results when the interfering agent is other nano-sized material (gold nanoparticles), and Fig. 14(D) shows detection results when the interfering agent is organic matter (humic acid). Experimental results confirmed that LFA has the ability to consistently display detection signals depending on the concentration of nanoplastics, despite the presence of these interfering agents. This suggests that the LFA methodology of the present disclosure can reliably detect specific nanoplastics without being affected by interfering agents.

### 8. Evaluation of Detection Ability in Actual Samples

The present disclosure aims to detect nanoplastics in various actual samples in order to evaluate the efficiency of a nanoplastic detection method that may be applied to an actual use environment. Actual samples selected for verification of the present disclosure included tap water, drinking water in polyethylene terephthalate (PET) bottles, sparkling water in PET bottles, water that passed through a filtered water purifier, and tea contained in tea bags.

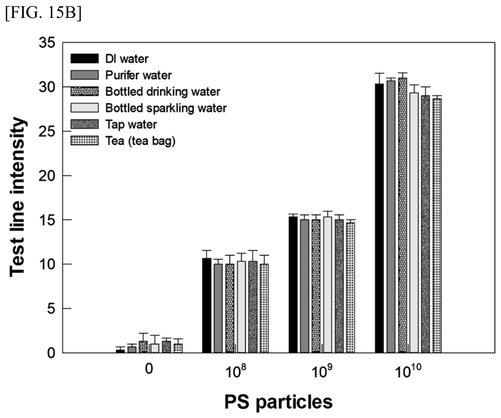

Fig. 15 includes images (A) and a graph (B), which show the results of detecting 100 nm polystyrene nanoplastics in each actual sample by LFA. In an example of the present disclosure, standardized concentrations (10⁸, 10⁹, and 10¹⁰ particles) of nanoplastics were added to each actual sample, and then the presence of nanoplastics was detected using LFA. The verification results confirmed that nanoplastics could be detected in all samples. This suggests that the nanoplastic detection method provided by the present disclosure has high detection ability even under various aquatic environmental conditions.

### 9. Validation of Portable Fluorescence Measurement Device

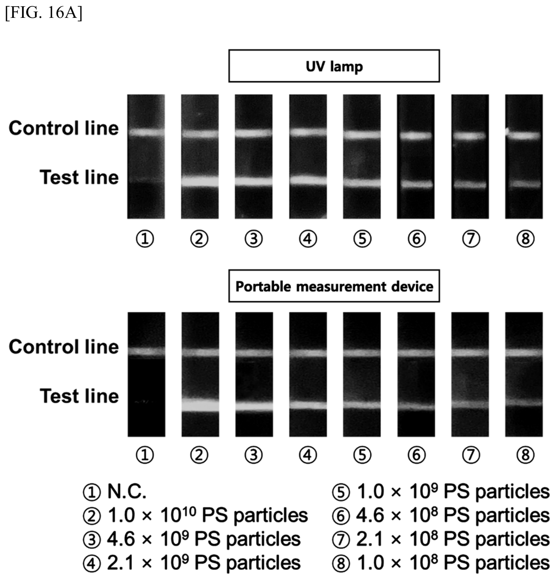

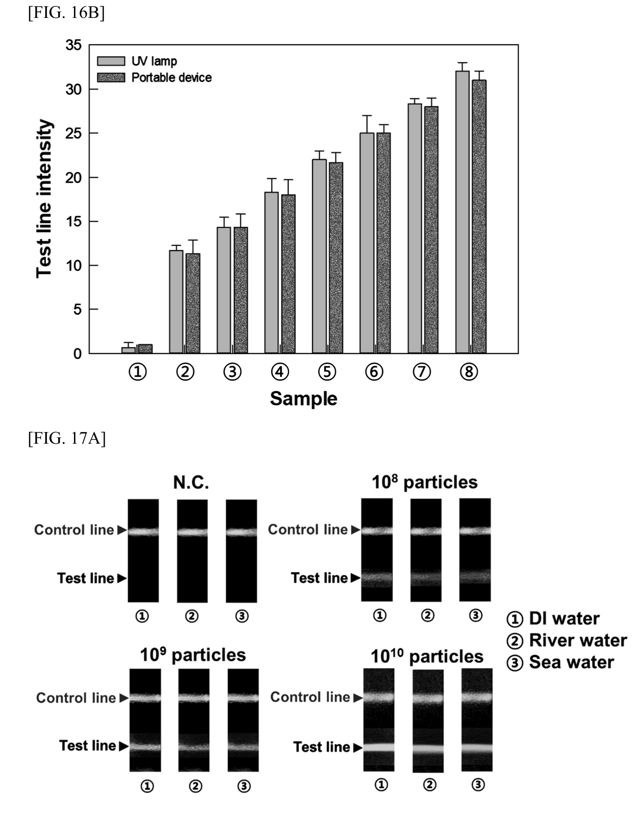

Fig. 16 shows images (A) and a graph (B) depicting the results of comparing the performance of a portable fluorescence measurement device and a laboratory fluorescence measurement device (UV lamp). The performance of the portable fluorescence measurement device and the laboratory fluorescence measurement device when detecting 100 nm polystyrene nanoplastics at various concentrations (10⁸, 10⁹, and 10¹⁰ particles) using them was compared. In this experiment, the detection abilities of a portable fluorescence measurement device that may be used immediately on-site and a traditional laboratory fluorescence measurement device used in a controlled environment were evaluated under identical conditions.

It was confirmed that the results of detecting 100 nm polystyrene nanoplastics at different concentrations were similar in both devices. These results demonstrate that the portable fluorescence measurement device provides accuracy and sensitivity equivalent to those of the laboratory device. The detection performance of the portable device demonstrates that the portable device can effectively identify nanoplastics and measure the concentration of nanoplastics compared to the laboratory-based device, thereby contributing to real-time data acquisition and rapid decision-making support on-site.

### 10. Evaluation of Ability to Detect Nanoplastics in Seawater and Freshwater Samples

The effectiveness of a nanoplastic detection method applicable to actual seawater and freshwater was evaluated. The goal was to detect nanoplastics in various environmental samples. A nanoplastic mixture sample was prepared by mixing five types of nanoplastics (polystyrene (PS), low-density polyethylene (LDPE), polypropylene (PP), polyvinyl chloride (PVC), and polyethylene terephthalate (PET)) in equal proportions.

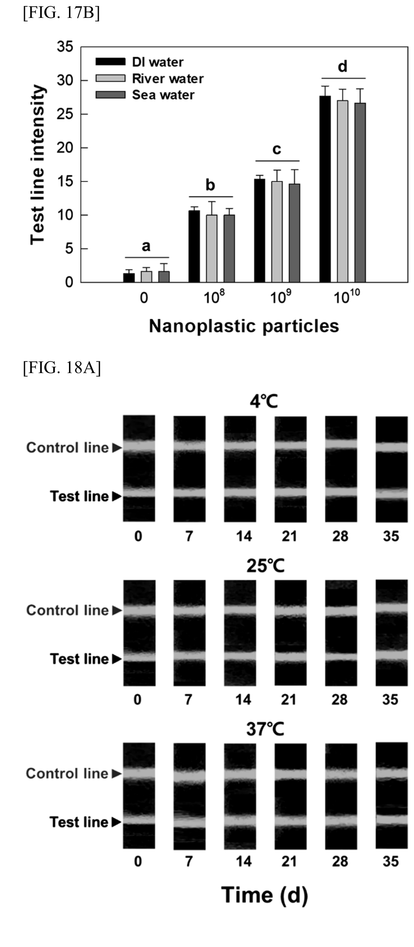

Fig. 17 shows images (A) and a graph (B) depicting the results of detecting nanoplastic mixture samples in each environmental sample by LFA. Nanoplastics at various concentrations (10⁸, 10⁹, and 10¹⁰ particles) were added to each actual sample, and then the presence of nanoplastics was detected using LFA. The verification results confirmed that nanoplastics could be detected in all samples. This suggests that the nanoplastic detection method provided by the present disclosure has high detection capabilities even under various aquatic environmental conditions.

### 11. Stability of LFA

To verify whether the LFA strip developed in the present disclosure maintains its consistent detection ability even after storage for a certain period of time under various environmental conditions, the performance stability of the LFA strip under various storage conditions was evaluated. To this end, the fabricated LFA strips were wrapped in aluminum foil to prevent light exposure, and then sealed in resealable zipper bags and stored under different temperature conditions of 4°C, 25°C, and 37°C. PBN was then prepared and applied to the conjugate pad immediately before nanoplastic detection.

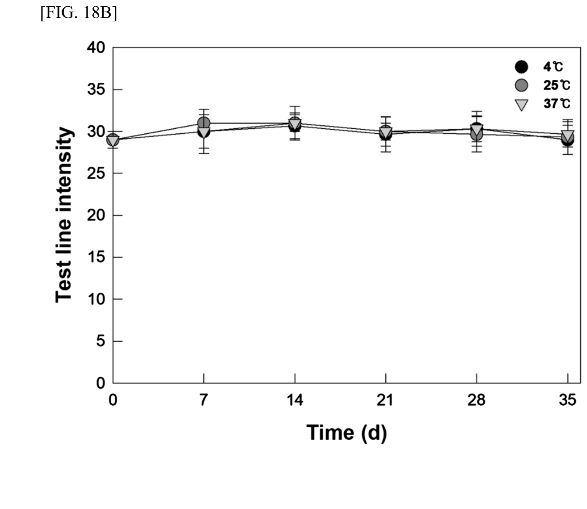

Fig. 18 shows images (A) and a graph (B) depicting the results of analyzing 10¹⁰ particles of 100 nm polystyrene nanoplastics using strips stored for a certain period of time under each storage condition. Strips were taken daily from each temperature condition and applied to samples, and then the fluorescence signal intensity was measured to evaluate changes in performance depending on the storage period. The measurement results confirmed that the strips maintained stable fluorescence signal intensities even after 35 days of storage under all temperature conditions. These results suggest that the LFA strip according to the present disclosure may be used stably without any degradation in detection performance even after long-term storage under appropriate storage conditions.

Although the present disclosure has been described above with reference to the embodiments, the present disclosure is not limited to the embodiments disclosed herein, and it is obvious that various modifications can be made by those skilled in the art within the scope of the technical idea of the present disclosure. In addition, although the effects according to the configuration of the present disclosure have not been explicitly described while describing the embodiments of the present disclosure, it is natural that the effects that can be predicted by the configuration should also be acknowledged.

## Claims

1. A sensor for detection of nanoplastics, comprising:
a membrane;
a sample pad, a conjugate pad and an absorbent pad sequentially arranged on the membrane; and
a test line and control line disposed between the conjugate pad and the absorbent pad,
wherein the conjugate pad comprises a fluorescent dye that binds to a target material,
a first polymer that binds to the target material is immobilized on the test line, and
a second polymer that binds to the fluorescent dye is immobilized on the control line.

2. The sensor of claim 1, wherein the test line and the control line are disposed on the membrane sequentially in a direction from the conjugate pad to the absorbent pad.

3. The sensor of claim 1 and/or claim 2, wherein the fluorescent dye comprises at least one selected from the group consisting of polyarylene ether-based fluorescent dyes, pyrene-based fluorescent dyes, stilbene-based fluorescent dyes, rhodamine-based fluorescent dyes, oxazone-based fluorescent dyes, and combinations thereof.

4. The sensor of any one of claims 1 to 3, wherein the fluorescent dye comprises at least one selected from the group consisting of tetraphenylethylene, 1-pyrenebutyric acid N-hydroxysuccinimidyl ester, 4-dimethylamino-4'-nitrostilbene, rhodamine B, Nile Red, Nile Blue, and combinations thereof.

5. The sensor of any one of claims 1 to 4, wherein the conjugate pad further comprises a surfactant.

6. The sensor of claim 5, wherein the surfactant comprises at least one selected from the group consisting of anionic surfactants, cationic surfactants, nonionic surfactants, amphoteric surfactants, and combinations thereof.

7. The sensor of claim 5 and/or claim 6, wherein the surfactant comprises at least one selected from the group consisting of sodium dodecyl sulfate, sodium lauryl sulfate, cetyltrimethylammonium bromide, Triton X-100, Tween-20, Tween-80, cocamidopropyl betaine, and combinations thereof.

8. The sensor of any one of claims 1 to 7, wherein the first polymer comprises at least one selected from the group consisting of synthetic polymers, naturally occurring polymers, conductive polymers, molecularly imprinted polymers, and combinations thereof.

9. The sensor of any one of claims 1 to 8, wherein the first polymer comprises at least one selected from the group consisting of polyacrylamide, polyethyleneimine, chitosan, polyaniline, polythiophene, and combinations thereof.

10. The sensor of any one of claims 1 to 9, wherein the membrane is manufactured to comprise at least one selected from the group consisting of nitrocellulose, polyethersulfone, polyethylene, nylon, polyvinylidene fluoride, polyester, polypropylene, and combinations thereof.

11. The sensor of any one of claims 1 to 10, wherein detection of the target material is performed by performing qualitative analysis to confirm the presence of the target material based on whether the test line develops color, and performing quantitative analysis to confirm the amount of the target material by measuring a signal of the fluorescent dye.

12. The sensor of any one of claims 1 to 11, wherein the second polymer comprises at least one selected from the group consisting of polyethylene, polyethylene terephthalate, polyvinyl chloride, polyamide, polypropylene, polystyrene, polycarbonate, and combinations thereof.

13. The sensor of any one of claims 1 to 12, wherein the sensor is based on lateral flow assay.

14. A method of detecting a target material using the sensor according to any one of claims 1 to 13, comprising steps of:
introducing a sample containing a target material into the sample pad; and
measuring a signal generated at the test line.

15. The method of claim 14, wherein detection of the target material is performed by performing qualitative analysis to confirm the presence of the target material based on whether the test line develops color, and performing quantitative analysis to confirm the amount of the target material by measuring a signal of the fluorescent dye.
